# EUROPEAN PATENT SPECIFICATION

(11) **EP 1 426 011 B1**
(45) Date of publication and mention of the grant of the patent: **14.02.2007**
(21) Application number: 03027817.0
(22) Date of filing: 03.12.2003
(51) Int. Cl.: A61B 8/08, A61B 17/34, A61B 10/00

(54) **Ultrasonic puncture needle**
Ultraschallpunktionsnadel
Aiguille de ponction ultrasonique

(30) Priority: 05.12.2002 JP 2002354289
(43) Date of publication of application: 09.06.2004
(73) Proprietor: Olympus Corporation, Shibuya-ku, Tokyo (JP)
(72) Inventor: Ichikawa, Yusuke, Hachioji-shi Tokyo (JP)
(74) Representative: von Hellfeld, Axel

(56) References cited:
- EP-A- 1 132 049
- US-A- 4 401 124
- US-A- 4 869 259
- US-A- 5 759 154
- US-A- 5 769 795

## Description

### BACKGROUND OF THE INVENTION

### 1. Field of the Invention

The present invention relates to an ultrasonic puncture needle used by being inserted into the body cavity for performing suctioning biopsy, performing an injection, or the like.

### 2. Description of the Related Art

Conventionally, methods are known wherein puncture needle pierces body tissue so as to reach an affected portion within the body cavity, which is to be examined under ultrasonic observation, so as to sample tissue within the body cavity or body fluid. In these methods, the aforementioned puncture needle is inserted into the portion which is to be examined, e.g., digestive organ walls such as the stomach, duodenum, or the like, and furthermore, deep internal organs such as the spleen, liver, kidney, or the like, while observing the body cavity using an ultrasonic endoscope.

With these methods, the puncture needle is required to be inserted with a suitable insertion depth. That is to say, in the event that the puncture needle is inserted with too great a depth or too small a depth, the tip of the puncture needle does not stop at the portion which is to be examine. In this case, sampling of tissue within the body cavity, body liquid, or the like, at a desired portion cannot be performed.

With conventionally-used ultrasonic puncture needles (which will be referred to as "puncture needles" hereafter), the ultrasonic images of the puncture needle, generated from ultrasonic waves reflected from the surface of the puncture needle, cannot be clearly displayed in ultrasonic observation images. The reason is that the puncture needle is formed with an outer diameter of less than 1 mm, and accordingly, sufficient reflection echoes do not occur due to reflection from the surface of the puncture needle, leading to unclear ultrasonic images of the puncture needle having a low contrast in ultrasonic observation images. Accordingly, the surgeon cannot obtain the precise information with regard to the position of the tip of the puncture needle as to the portion which is to be examined, and the distance therebetween.

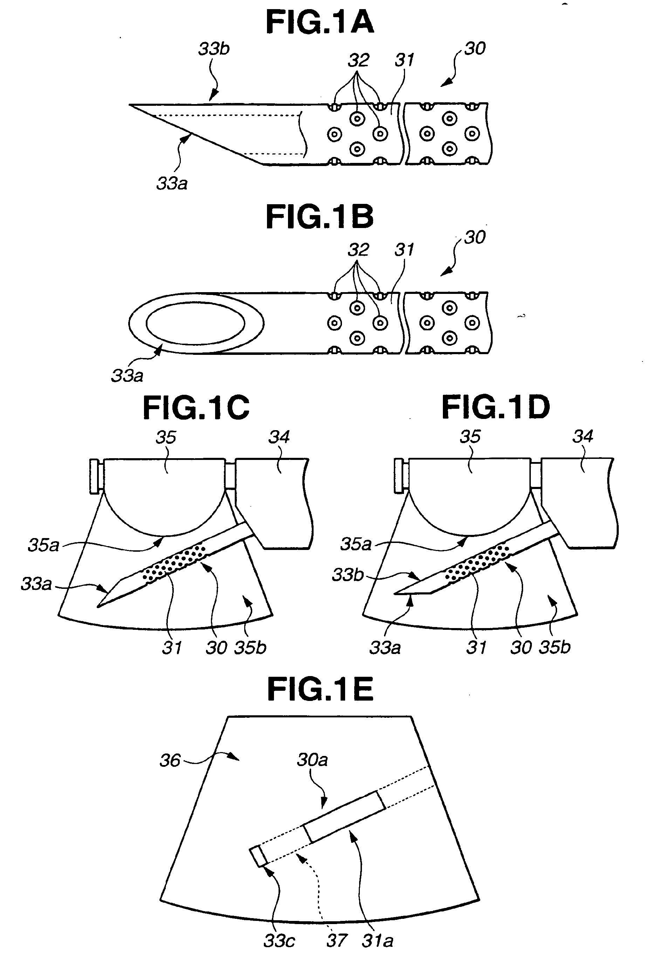

In order to solve the above-described problem, an arrangement has been disclosed in Japanese Unexamined Patent Application Publication No. 2003-190179, which includes a needle tube 30 in the shape of a pipe having a configuration as shown in Figs. 1A and 1B. The needle tube 30 includes multiple staggered-array doughnut-shaped recesses 32 formed at predetermined positions on the surface of the tip portion 31 thereof. Thus, in the event that the ultrasonic waves are cast onto the needle tube 30 from directions perpendicular to the longitudinal direction of the needle tube 30, or even in the event the ultrasonic waves are cast from directions other than the perpendicular directions, the ultrasonic waves are reflected with great intensity in the incident direction, and thus the images of the needle tube are clearly displayed in the ultrasonic observation images.

Then, in the event that the surgeon protrudes the needle tube 30 from a treatment tool insertion channel (not shown) of an ultrasonic endoscope 34 with the positional relationship between the needle tube 30 and the ultrasonic endoscope 34 being such that a cutting-tip portion 33a faces the scanning face 35a of an ultrasonic transducer 35 within the ultrasonic scanning range 35b, as shown in Fig. 1C, it is checked that an image of the tip portion of the needle tube and the end of the cutting-tip portion is displayed in the ultrasonic observation image.

However, in the event that the surgeon protrudes the needle tube 30 from the unshown treatment tool insertion channel of the ultrasonic endoscope 34 with the positional relation between the needle tube 30 and the ultrasonic endoscope 34 being such that the back-of-cutting-tip portion 33b faces the scanning face 35a of the ultrasonic transducer 35 within the ultrasonic scanning range 35b, as shown in Fig. 1D, a needle tube image 30a is displayed in the ultrasonic observation image 36 as shown in Fig. 1E. As shown in Fig. 1E, an unimaged portion 37 denoted by broken lines occurs between a cutting-tip-side image 33c with a narrow width representing the tip of the cutting-tip portion 33a, and a needle-tube-surface image 31a representing the surface of the tip portion 31 including the doughnut-shaped recesses, in the needle tube image 30a.

In the event that the surgeon inserts the needle tube, which creates an image such as the needle tube image 30a in the ultrasonic observation image 36, into a small-sized affected portion near the wall of the body cavity, two problems occur. One is that the needle tube is protruded with a short length, and accordingly, the needle-tube-surface image 31a on the rear side of the unimaged portion 37 cannot be observed. The other is that the wall of the body cavity is imaged with a great intensity due to a high echo density, leading to difficulty in observing the cutting-tip-side image 33c. As a result, these problem leads to a disadvantageous situation wherein the surgeon cannot easily obtain the information with regard to the positional relation between the tube needle and the tissue within the body cavity at the time of insertion of the puncture needle.

US Patent No. 5,759,154 discloses a solid or tubular needle according to the preamble of claim 1, having square-shaped recesses formed by masking and etching a solid rod. The tip of the rod is then formed into an angled cutting surface. US Patent No. 4,869,259 describes a needle, the tip of which is roughened by particle-blasting to produce an irregular surface topography. US Patent No. 4,401,124 relates to a surgical needle having helical threads cut into its outer surface at a tip portion thereof.

Accordingly, it is an object of the present invention to provide an ultrasonic puncture needle which creates a needle tube image with a small-sized unimaged portion in an ultrasonic observation image so that the surgeon can easily insert the puncture needle into the portion which is to be examined, in a sure manner.

### SUMMARY OF THE INVENTION

The present invention provides an ultrasonic puncture needle as recited in claim 1. Preferred features of the invention are recited in dependent claims 2 to 4.

An ultrasonic puncture needle according to the present invention comprises a needle tube which is to be inserted into a treatment tool insertion channel of an ultrasonic endoscope for insertion into tissue within a body cavity. The needle tube comprises a tube portion having a distal tip portion, the tube portion including multiple staggered-array doughnut-shaped recesses over a predetermined range on the surface of the tip portion of the needle tube from the portion near the tip of the needle tube. Accordingly, a needle tube image with small-sized unimaged portion is generated in an ultrasonic observation image. Thus, the surgeon can perform insertion of the puncture needle into the portion which is to be examined, in a sure manner. The ultrasonic puncture needle may also include a sheath, through which the needle tube is inserted into the body cavity.

The above and other objects, features and advantages of the invention will become more clearly understood from the following description referring to the accompanying drawings.

### BRIEF DESCRIPTION OF THE DRAWINGS

Fig. 1A is a side view for describing the configuration of the tip portion of a conventional needle tube;
Fig. 1B is a bottom view for describing the configuration of the tip portion of a conventional needle tube;
Fig. 1C is a diagram for describing the positional relation between the needle tube and the ultrasonic endoscope, wherein the cutting-tip portion faces the scanning face of the ultrasonic transducer, at the time of the surgeon protruding the conventional needle tube from the treatment tool insertion channel of the ultrasonic endoscope.
Fig. 1D is a diagram for describing the positional relation between the needle tube and the ultrasonic endoscope, wherein the back of the cutting-tip portion faces the scanning face of the ultrasonic transducer, at the time of the surgeon protruding the conventional needle tube from the treatment tool insertion channel of the ultrasonic endoscope.
Fig. 1E is a diagram for describing a needle tube image in an ultrasonic observation image generated due to the conventional needle tube in the positional relation wherein the back of the cutting-tip portion of the conventional needle tube faces the scanning face of the ultrasonic transducer.

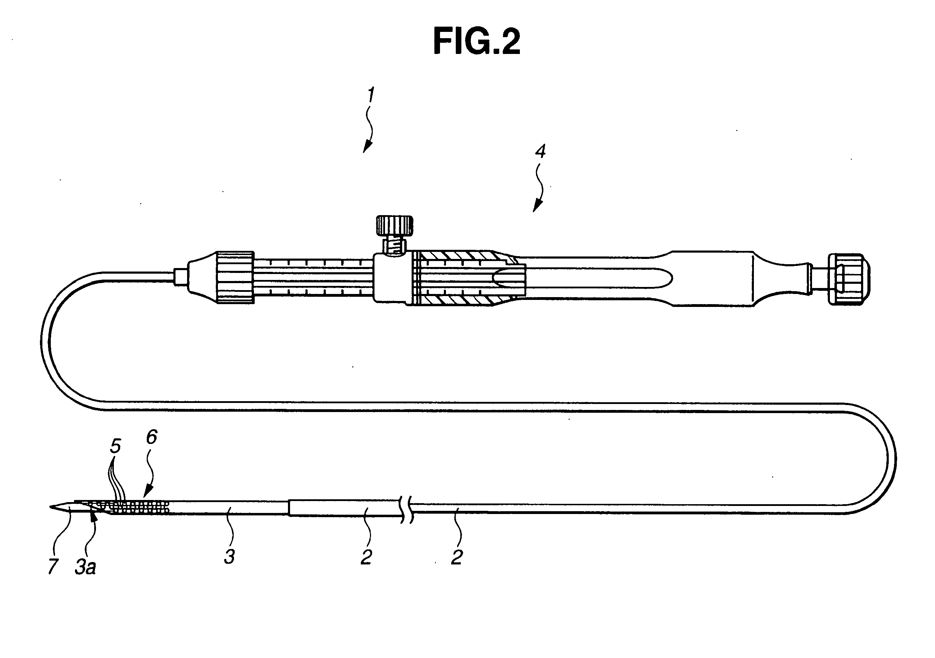

Fig. 2 is a diagram for describing an ultrasonic puncture needle;
Fig. 3A is a side view of the tip portion of a needle tube according to the present invention;
Fig. 3B is a top view of the tip portion of the needle tube according to the present invention;
Fig. 3C is a bottom view of the tip portion of the needle tube according to the present invention;
Fig. 4 is a schematic drawing showing the needle tube in an unfurled manner, for describing an array pattern of doughnut-shaped recesses included on the tip portion of the needle tube;
Fig. 5 is a cross-sectional view taken along line 4-4 in Fig. 3A;
Fig. 6A is a diagram which shows the positional relation between the needle tube and an ultrasonic endoscope, wherein the back of a cutting-tip portion of the needle tube faces the scanning face of an ultrasonic transducer, at the time of the surgeon protruding the needle tube of the ultrasonic puncture needle from a treatment tool insertion channel of the ultrasonic endoscope;
Fig. 6B is a diagram which shows an ultrasonic image generated in the positional relation shown in Fig. 6A;
Fig. 7A is a diagram which shows the positional relation between the needle tube and the ultrasonic endoscope, wherein the side of the cutting-tip portion of the needle tube faces the scanning face of the ultrasonic transducer, at the time of the surgeon protruding the needle tube of the ultrasonic puncture needle from the treatment tool insertion channel of the ultrasonic endoscope;
Fig. 7B is a diagram which shows an ultrasonic image generated in the positional relation shown in Fig. 7A;
Fig. 8A is a diagram which shows the positional relation between the needle tube and the ultrasonic endoscope, wherein the cutting-tip portion of the needle tube faces the scanning face of the ultrasonic transducer, at the time of the surgeon protruding the needle tube of the ultrasonic puncture needle from the treatment tool insertion channel of the ultrasonic endoscope; and
Fig. 8B is a diagram which shows an ultrasonic image generated in the positional relation shown in Fig. 8A.

### DETAILED DESCRIPTION OF THE PREFERRED EMBODIMENTS

Description will be made below regarding embodiments of the present invention with reference to the drawings.

As shown in the drawings, an ultrasonic puncture needle 1 according to the present embodiment principally comprises a sheath 2, a needle tube 3, and an operating portion 4.

The aforementioned sheath 2 is inserted into a treatment tool insertion channel of an endoscope, for example. The aforementioned operating portion 4 serves as a holding portion, as well, and is disposed at the base portion of the aforementioned sheath 2. The needle tube 3 is formed of a slender stainless pipe with a small tube thickness, for example. The needle tube 3 includes a puncturing portion 6 having a sharp cutting-tip portion 3a or the like, at the tip thereof. The needle tube 3 is slidably disposed within the sheath 2 through the operating portion 4.

Note that an arrangement may be made wherein a stylette 7 having a sharp-shaped tip is detachably disposed within the through hole of the aforementioned needle tube 3.

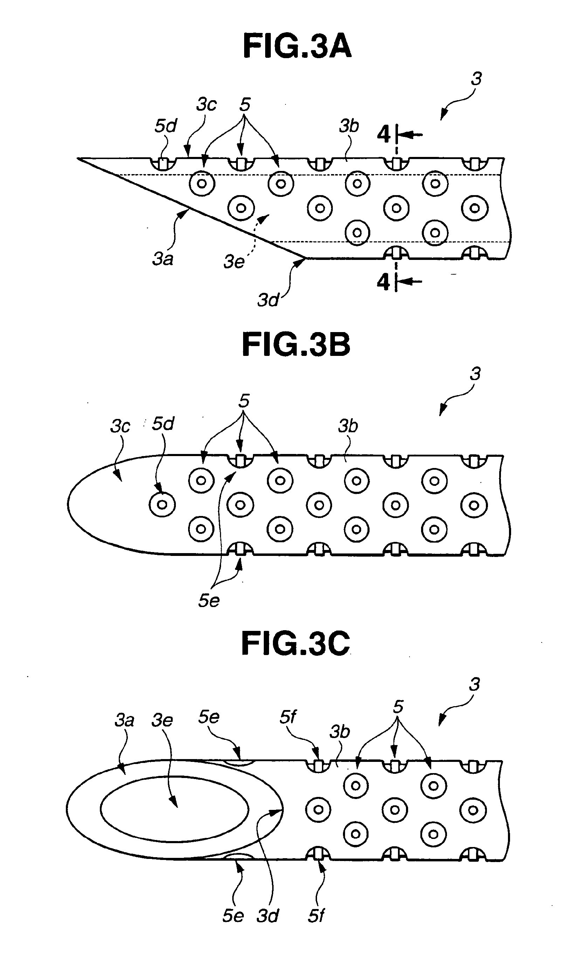

Next, description will be made regarding the tip portion of the needle tube 3 with reference to Figs. 3A through 5.

As shown in Figs. 3A through 4, a tube portion 3b forming the puncturing portion 6 provided in a predetermined range from the portion near the tip of the aforementioned needle tube 3 includes multiple staggered-array doughnut-shaped recesses 5 on the surface thereof, serving as so-called ultrasonic wave reflection means for reflecting ultrasonic waves. The multiple doughnut-shaped recesses 5 are formed so as to not have adverse effects upon the aforementioned cutting-tip portion 3a. These multiple doughnut-shaped recesses 5 are formed on the tip side of the tube portion 3b with a great density from a predetermined portion on a back-of-cutting-tip portion 3c on the back side of the aforementioned cutting-tip portion 3a so as to be formed on the tube portion 3b in a radial pattern. The aforementioned multiple staggered-array doughnut-shaped recesses 5 are formed on the tube portion 3b using a YAG laser apparatus or an electric discharge machining apparatus under predetermined control set so as to form a staggered-array pattern.

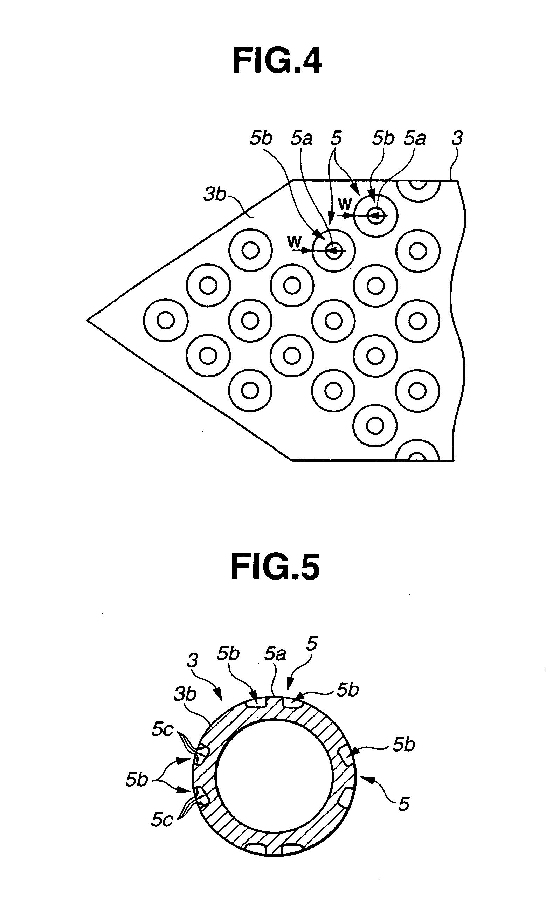

As shown in Fig. 5, the aforementioned doughnut-shaped recesses 5 are formed with the faces 5c of the bottoms and the sides, each generally flat in the cross-sectional view thereof, so as to obtain reflection echoes with a great intensity for the incident ultrasonic waves with a shallow incident angle or the like. These doughnut-shaped recesses 5 are formed using the YAG laser with a laser beam spot diameter set to 0.1 mm under positioning control for the laser beam and the needle tube 3, for example. Specifically, with regard to each doughnut-shaped recess, a ring-shaped recess 5b with a predetermined width (W) formed at a predetermined position on the needle tube 3, whereby the center portion thereof remain as protrusion 5a with a predetermined size, as shown in Figs. 4 and 5.

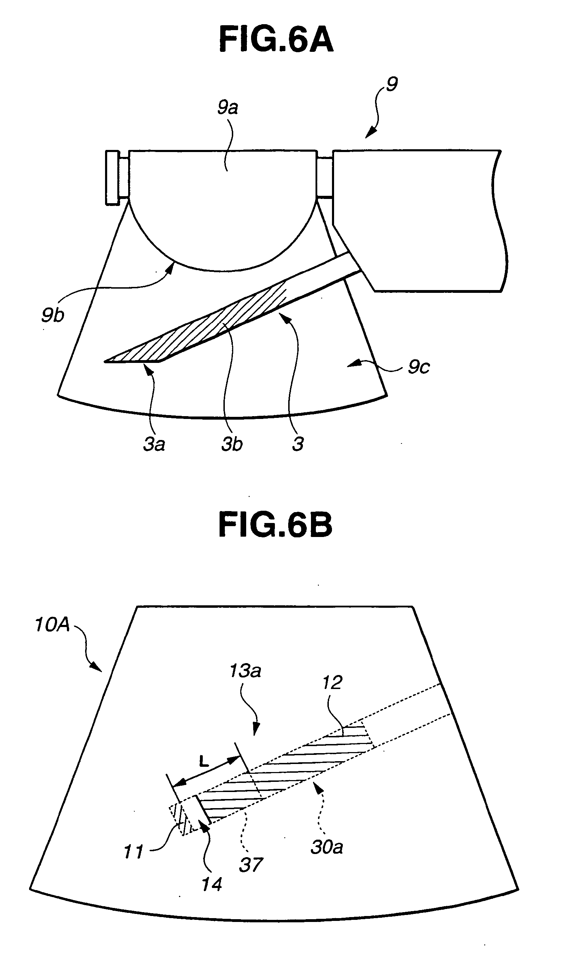

Next, description will be made regarding the operation of the ultrasonic puncture needle 1 having the above-described configuration in a case of the surgeon protruding the needle tube 3 thereof from the treatment tool insertion channel of the ultrasonic endoscope, with reference to Figs. 6A through 8B.

First, as shown in Fig. 6A, the needle tube 3 is protruded from an unshown treatment tool insertion channel of an ultrasonic endoscope 9 so as to be positioned within the ultrasonic scanning range 9c of an ultrasonic transducer 9a. Now, let us say that the back-of-cutting-tip portion 3c of the needle tube 3 faces in the direction of the ultrasonic transducer 9a. In this case, as shown in the top view in Fig. 3b, the doughnut-shaped recesses 5 formed on the tube portion 3b in the range from the doughnut-shaped recess 5 at the base thereof up to the first doughnut-shaped recess 5d at the tip thereof face the scanning face 9b of the ultrasonic transducer 9a.

Thus, as shown in Fig. 6B, a needle tube image 13a is displayed in an ultrasonic observation image 10A. The needle tube image 13a contains a needle-tube-tip image 11 due to the ultrasonic waves reflected from the tip of the cutting-tip portion 3a and a needle-tube-surface image 12 representing the tube portion 3b due to the ultrasonic waves reflected from the doughnut-shaped recesses 5 formed on the back-of-cutting-tip portion 3c of the needle tube 3 in a predetermined range from the portion near the tip thereof.

The aforementioned needle tube image 13a forms an ultrasonic image representing the generally entire needle tube from the tube portion 3b including the doughnut-shaped recesses 5 up to the tip of the needle tube. However, more precisely, an unimaged portion 14 occurs between the needle-tube-tip image 11 and the needle-tube-surface image 12. The aforementioned unimaged portion 14 occurs due to the portion having no doughnut-shaped recesses between the tip of the needle tube and the first doughnut-shaped recess 5d formed at the head thereof shown in the top view in Fig. 3B. However, the unimaged portion 14 occurs with a length markedly shorter than the length L of an unimaged portion 37 occurring in a needle tube image 30a generated due to the conventional needle tube 30 denoted by broken lines.

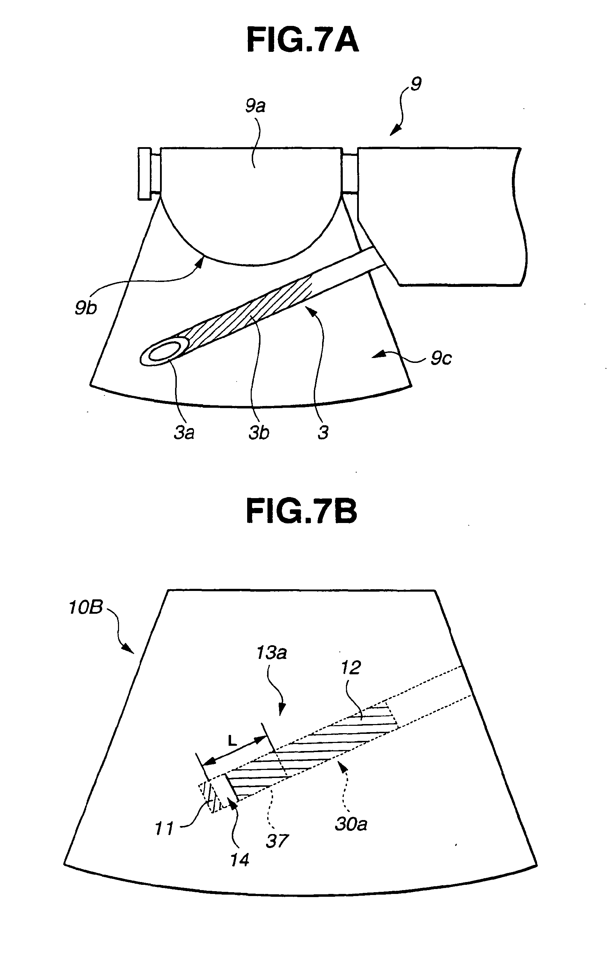

Next, the needle tube 3 is protruded from the unshown treatment tool insertion channel of the ultrasonic endoscope 9 so as to be positioned within the ultrasonic scanning range 9c of the ultrasonic transducer 9a as shown in Fig. 7A. Now, let us say that one side of the cutting-tip portion 3a of the needle tube 3 faces in the direction of the aforementioned ultrasonic transducer 9a. In this case, the doughnut-shaped recesses 5, formed on the tube portion 3b, including the sides of the doughnut-shaped recesses 5 in the range from the doughnut-shaped recess 5 at the base of the tube portion 3b up to the first doughnut-shaped recess 5d at the head thereof, face the scanning face 9b of the aforementioned ultrasonic transducer 9a, as shown in the side view in Fig. 3A.

Accordingly, while the aforementioned scanning face 9b and the doughnut-shaped recesses 5 face each other in a manner somewhat different from the relation in the above-described case shown in Fig. 6A, the doughnut-shaped recesses 5 in the range from the doughnut-shaped recess 5 at the base of the needle tube 3 up to the first doughnut-shaped recess 5d at the head thereof face the scanning face 9b generally in the same way.

Thus, as shown in Fig. 7B, the needle-tube image 13a is displayed in an ultrasonic observation image 10B, which contains the needle-tube-tip image 11 and the needle-tube-surface image 12 with the aforementioned unimaged portion 14 therebetween, generally in the same way as shown in Fig. 6B described above. The unimaged portion 14 occurs with a length markedly shorter than the length L of the unimaged portion 37 occurring in the needle tube image 30a generated due to the conventional needle tube 30 denoted by broken lines.

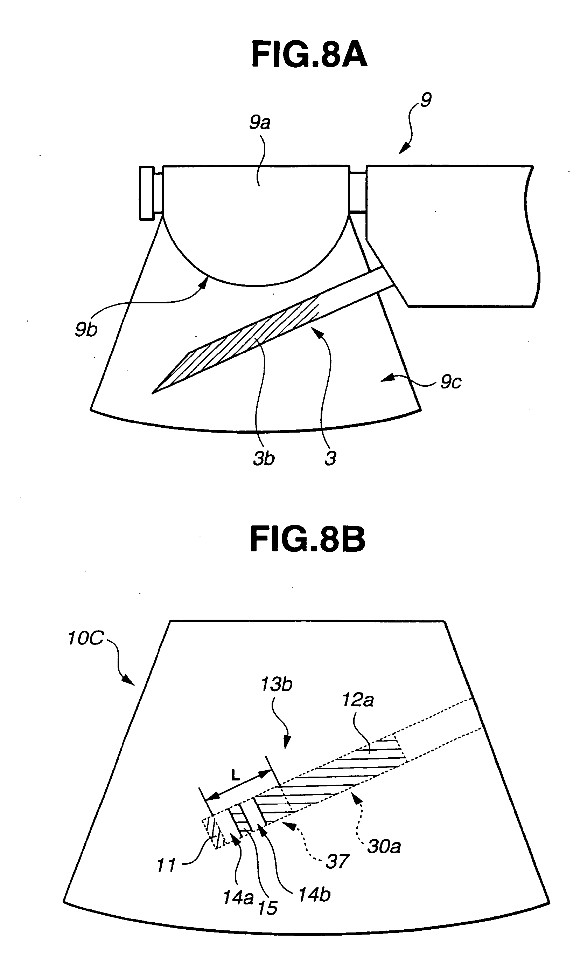

Next, the needle tube 3 is protruded from the unshown treatment tool insertion channel of the ultrasonic endoscope 9 so as to be positioned within the ultrasonic scanning range 9c of the ultrasonic transducer 9a as shown in Fig. 8A. Now, let us say that the cutting-tip portion 3a of the needle tube 3 faces the aforementioned ultrasonic transducer 9a. In this case, the cutting-tip portion 3a formed of an inclined face having a through hole 3e, the sides of doughnut-shaped recesses 5e formed on the side face of the cutting-tip portion 3a, and the tube portion 3b including the doughnut-shaped recesses 5 formed on the rear side of the base of the cutting-tip portion 3a, face the scanning face 9b of the ultrasonic transducer 9a, as shown in the bottom view in Fig. 3C.

Thus, a needle-tube image 13b is displayed in an ultrasonic observation image 10C as shown in Fig. 8B. The needle tube image 13b contains the needle-tube-tip image 11 generated due to reflection of the ultrasonic waves from the tip of the cutting-tip portion 3a, a needle-tube-rear-portion image 15 generated due to reflection of the ultrasonic waves from an edge rear portion 3d of the cutting-tip portion 3a, and a needle-tube-surface image 12a generated due to reflection of the ultrasonic waves from the multiple doughnut-shaped recesses 5 formed on the rear side of the cutting-tip portion 3a.

While the aforementioned needle tube image 13b is an ultrasonic image which represents the generally entire needle tube in the range from the tube portion 3b including the doughnut-shaped recesses 5 up to the tip of the needle tube, an unimaged portion 14a occurs between the needle-tube-tip image 11 and the needle-tube-rear-portion image 15, as well as unimaged portion 14b occurring between the needle-tube-rear-portion image 15 and the needle-tube-surface image 12.

The aforementioned unimaged portion 14a occurs due to the portion between the tip of the needle portion 3a and the edge rear portion 3d, shown in the bottom view in Fig. 3C. On the other hand, the aforementioned unimaged portion 14b occurs due to the portion between the edge rear portion 3d and doughnut-shaped recesses 5f formed on the rear side of the cutting-tip portion 3a, shown in the bottom view in Fig. 3C. While such unimaged portions 14a and 14b occur in the needle tube image 13b, these unimaged portions 14a and 14b occurs with lengths markedly shorter than the length L of the unimaged portion 37 occurring in the needle tube image 30a generated due to the conventional needle tube 30 denoted by broken lines.

As described above, with the ultrasonic puncture needle according to the present invention, the multiple staggered-array doughnut-shaped recesses are formed so as to be spread with a great density over a predetermined range in a radial pattern from the portion near the tip of the needle tube, and accordingly, the ultrasonic waves output from the ultrasonic transducer are reflected from the needle tube with a greater intensity and free of large portions where the ultrasonic waves are not reflected, thereby displaying a clear needle-tube image without large-sized unimaged portions on an ultrasonic observation image.

Furthermore, with the ultrasonic puncture needle according to the present invention, the doughnut-shaped recesses are formed with a great density in a predetermined pattern such that the doughnut-shaped recesses do not have adverse effects upon the insertion performance of the needle tube.

Thus, with the ultrasonic puncture needle according to the present invention, the surgeon can perform precise measurement of the positioning relation and the distance between the needle tube and the portion which is to be examined, and also can perform insertion of the needle tube in a sure manner, even in the event that the surgeon performs insertion of the needle tube into a small-sized affected portion near the wall of the body cavity.

Having described the preferred embodiments of the invention referring to the accompanying drawings, it should be understood that the present invention is not limited to those precise embodiments and various changes and modifications thereof could be made by one skilled in the art without departing from the scope of the invention as defined in the appended claims.

## Claims

1. An ultrasonic puncture needle (1) comprising a needle tube (3) which is to be inserted into a treatment tool insertion channel of an ultrasonic endoscope so as to be inserted into tissue within a body cavity, wherein the needle tube (3) includes a plurality of recesses (5) over a predetermined range on a surface (3c) of a tip portion thereof from a tip thereof on a back side of a cutting-tip portion, **characterized in that** the plurality of recesses (5) are formed at positions such that overlap of the recesses (5) and the cutting-tip portion (3a) of the needle tube (3) does not occur.

2. An ultrasonic puncture needle (1) according to claim 1, wherein the plurality of recesses (5) are arrayed so as to be spread in a radial pattern from the tip of the needle tube (3).

3. An ultrasonic puncture needle(1) according to claim 1 or claim 2, wherein the plurality of recesses (5) are formed in a doughnut shape using a laser apparatus or an electric discharge machining apparatus.

4. An ultrasonic puncture needle (1) according to any one of claims 1 to 3, further comprising a sheath (2) to be inserted into the treatment tool insertion channel of the ultrasound endoscope and through which the needle tube (3) is inserted.

## Patentansprüche

1. Ultraschall-Durchstossnadel (1), aufweisend eine Nadelröhre (3), die in einen Behandlungswerkzeugeinführkanal eines Ultraschallendoskops einzuführen ist, um so in Gewebe einer Körperhöhle eingeführt zu werden, wobei die Nadelröhre (3) eine Mehrzahl von Aussparungen (5) über einen vorbestimmten Bereich an einer Oberfläche (3c) eines zugehörigen Spitzenteils von einer zugehörigen Spitze an einer Rückseite eines Schneidspitzenteils umfasst,
**dadurch gekennzeichnet, dass** die Mehrzahl der Aussparungen (5) an solchen Orten ausgebildet sind, dass keine Überlappung der Aussparungen (5) und des Schneidspitzenteils (3a) der Nadelröhre (3) auftritt.

2. Ultraschall-Durchstossnadel (1) nach Anspruch 1, bei der die Mehrzahl der Aussparungen (5) so angeordnet ist, dass sie sich in einem radialen Muster von der Spitze der Nadelröhre (3) aus erstrecken.

3. Ultraschall-Durchstossnadel (1) nach Anspruch 1 oder 2, bei der die Mehrzahl der Aussparungen (5) unter Verwendung einer Laservorrichtung oder einer Funkenerosionsvorrichtung in einer Ringröhrenform ausgebildet ist.

4. Ultraschall-Durchstossnadel (1) nach einem der Ansprüche 1 bis 3, ferner aufweisend eine Hülle (2), die in den Behandlungswerkzeugeinführkanal des Ultraschallendoskops einzuführen ist, und durch welche die Nadelröhre (3) eingeführt wird.

## Revendications

1. Aiguille de ponction ultrasonique (1) comprenant un tube d'aiguille (3) qui doit être inséré dans un canal d'insertion d'outil de traitement d'un endoscope ultrasonique de façon à être inséré dans un tissu à l'intérieur d'une cavité corporelle, dans laquelle le tube d'aiguille (3) comprend une pluralité d'évidements (5) sur une étendue prédéterminée sur une surface (3c) d'une partie de bout de celui-ci à partir d'un bout de celui-ci sur un côté arrière d'une partie de bout coupant, **caractérisée en ce que** la pluralité d'évidements (5) sont formés à des positions telles qu'un chevauchement des évidements (5) et de la partie de bout coupant (3a) du tube d'aiguille (3) n'a pas lieu.

2. Aiguille de ponction ultrasonique (1) selon la revendication 1, dans laquelle la pluralité d'évidements (5) sont disposés de manière à être étalés dans une configuration radiale à partir du bout du tube d'aiguille (3).

3. Aiguille de ponction ultrasonique (1) selon la revendication 1 ou la revendication 2, dans laquelle la pluralité d'évidements (5) sont formés en forme d'anneau en utilisant un appareil à laser ou un appareil d'usinage à décharge électrique.

4. Aiguille de ponction ultrasonique (1) selon l'une quelconque des revendications 1 à 3, comprenant en outre une gaine (2) devant être insérée dans le canal d'insertion d'outil de traitement de l'endoscope ultrasonique et à travers laquelle le tube d'aiguille (3) est inséré.
